# EUROPEAN PATENT SPECIFICATION

(11) **EP 2 986 273 B1**
(45) Date of publication and mention of the grant of the patent: **21.02.2018**
(21) Application number: 14718563.1
(22) Date of filing: 15.04.2014
(51) Int. Cl.: A61K 8/39, A61K 8/44, A61Q 5/04, A61K 8/60, A61K 8/67, A61K 8/19, A61K 8/22, A61K 8/41, A61K 8/23, A61K 8/46, A61K 8/04

(54) **COSMETIC HAIRCARE COMPOSITION FOR RELAXING CURLS AND/OR FOR REDUCING VOLUME**
KOSMETISCHE HAARBEHANDLUNG ZUM ENTSPANNEN VON LOCKEN UND/ODER HAARVOLUMEN REDUKTION BEWIRKEN
COMPOSITION CAPILLAIRE POUR DEFRISER LES CHEVEUX ET/OU REDUIRE LEUR VOLUME

(30) Priority: 19.04.2013 FR 1353593; 19.04.2013 FR 1353595
(43) Date of publication of application: 24.02.2016
(73) Proprietor: L'Oréal, 75008 Paris (FR)
(72) Inventor: MELLUL, Myriam, F-75014 Paris (FR); PAUL, Laurence, F-95320 Saint Leu la Forêt (FR); LIVOREIL-DJAMPOU, Aude, F-93350 Le Bourget (FR)
(74) Representative: Casalonga
(86) International application number: PCT/EP2014/057647
(87) International publication number: WO 2014/170336

(56) References cited:
- FR-A1- 2 923 713
- GB-A- 2 068 031

## Description

The present invention relates to a composition for the cosmetic treatment of keratin fibres, which is intended in particular for relaxing the curls and/or reducing the volume of the said fibres and which comprises at least one amphoteric or zwitterionic surfactant, at least one cationic surfactant, at least one sulfureous reducing agent and at least one alkaline agent.

The present invention also relates to a cosmetic process for treating keratin fibres such as the hair, using the said composition.

According to a particular embodiment, this process consists in relaxing the curls of and/or in straightening keratin fibres, and comprises a step of applying to the keratin fibres the composition according to the invention, followed by a step of applying to the keratin fibres a second composition comprising one or more oxidizing agents and/or one or more reductones.

The invention also relates to the use of this composition for relaxing the curls and/or reducing the volume of keratin fibres.

Finally, a subject of the invention is a multi-compartment device or "kit" that is suitable for performing the process according to the invention.

To obtain permanent reshaping of the hair such as straightening of the hair, uncurling or relaxing of the curls, the technique most commonly used consists, in a first stage, in opening the -S-S- disulfide bonds of keratin (keratocystine) generally by means of a basic composition containing a sulfureous reducing agent (reduction step), and then, after having rinsed the head of hair thus treated, generally with water, in reconstituting, in a second stage, the said disulfide bonds by applying to the hair, which has been placed under tension beforehand, an oxidizing composition (oxidation step, also known as the fixing step) so as finally to give the hair the desired shape.

The new shape given to the hair by such a chemical treatment is eminently long-lasting and especially withstands washing with water or shampoos, as opposed to the simple standard techniques of temporary reshaping, such as hairsetting.

Many products intended for straightening or uncurling the hair or for relaxing curls exist on the market.

Products intended for straightening or uncurling the hair are generally formulated with thiols and alkaline agents, whereas products intended for relaxing curls tend rather to contain products such as cysteine and derivatives thereof.

These products are generally applied to curly or voluminous hair in order to obtain more or less pronounced straightening and a reduction in the volume and mass of the head of hair.

The application of these products is generally long, with a longer or shorter leave-on time depending on the product, the type of hair and the desired effect. It requires precise know-how.

Furthermore, reducing agents are generally used in high concentrations, which may lead to more or less pronounced degradation of the hair fibre, in particular when these fibres are coloured.

In addition, the use of such a straightening treatment is a choice that is generally drastic for the consumer, who cannot regulate the level of straightening at will.

Finally, the use of such a straightening treatment generally gives rise to odour problems that may prove to be troublesome to the consumer.

There is thus a real need to develop hair straightening treatments, which are lighter than those existing in the prior art, which not only degrade the hair fibre less, but also are easy to apply, and allow modulation of the straightening effect, for example by making use of repeated applications.

The Applicant has now discovered that the introduction of certain sulfureous active agents into a particular care base makes it possible to satisfy the abovementioned objectives.

In particular, it has discovered that by introducing at least one sulfureous reducing agent and at least one alkaline agent into a care base containing a particular combination of surfactants based on an amphoteric or zwitterionic surfactant, and a cationic surfactant, while respecting a particular weight concentration ratio between these two types of surfactants, a care composition having straightening activity on the hair may be obtained.

One subject of the present invention is thus a cosmetic composition comprising:
a) one or more amphoteric or zwitterionic surfactants,
b) one or more cationic surfactants,
c) one or more sulfureous reducing agents, and
d) one or more alkaline agents,
the weight ratio between the total amount of the amphoteric or zwitterionic surfactant(s), on the one hand, and the total amount of the cationic surfactant(s), on the other hand, being greater than or equal to 1.2.

The application of such a composition to keratin fibres is rapid and simple, and does not require any particular know-how.

Furthermore, the application of this composition makes it possible to obtain a relaxation of curls and/or a reduction of the volume of the head of hair that is gradual. In particular, it is possible to modulate the desired effect, by successive applications of such a composition.

In particular, the composition according to the invention may be used as a straightening maintenance product, especially between two straightening operations.

In addition, the composition according to the invention has good detergency properties and very good working qualities.

Finally, the treatment by means of the composition according to the invention virtually does not degrade hair fibres.

The invention also relates to a cosmetic process for treating keratin fibres, comprising the application to the said wet or dry fibres, in one or more stages, of the composition according to the invention.

According to a particular embodiment, the invention relates to a process for relaxing the curls of and/or for straightening keratin fibres, such as the hair, and which comprises:
a) a step of applying to the keratin fibres the composition according to the invention; followed by
b) a step of applying to the keratin fibres a second composition (B) comprising one or more oxidizing agents and/or one or more reductones.

The implementation of such a process is rapid and simple, and does not require any particular know-how.

Furthermore, the implementation of this process makes it possible to obtain a relaxation of curls and/or a reduction of the volume of the head of hair that is gradual. In particular, it is possible to modulate the desired effect, by successive implementations of such a process.

In particular, this process may be performed in order to maintain the straightening, especially between two straightening operations.

Also, the implementation of the process according to the invention does not degrade hair fibres.

Finally, the process according to the invention makes it possible to substantially reduce the unpleasant odours derived from performing a standard curl relaxation and/or straightening process.

Other characteristics and advantages of the invention will emerge more clearly on reading the description and the examples that follow.

The composition according to the invention advantageously comprises water or a mixture of water and of one or more cosmetically acceptable solvents chosen from C1-C4 lower alcohols, such as ethanol, isopropanol, tert-butanol or n-butanol, polyols such as propylene glycol, polyol ethers, C5-C10 alkanes, C3-C4 ketones, such as acetone and methyl ethyl ketone, C1-C4 alkyl acetates, such as methyl acetate, ethyl acetate and butyl acetate, dimethoxyethane and diethoxyethane, and mixtures thereof.

The amphoteric or zwitterionic surfactant(s) that are used in the composition according to the invention are the compounds of structures (II)

Ra'-C(o)-NHCH₂CH₂-N(B)(B') (II)

in which formula (II):
B represents the group -CH₂-CH₂-O-X';
B' represents the group -(CH₂)_{z}Y', with z = 1 or 2;
X' represents the group -CH₂-COOH, CH₂-COOZ', -CH₂CH₂-COOH or -CH₂CH₂-COOZ', or a hydrogen atom;
Y' represents the group -COOH, -COOZ', CH₂CH(OH)SO₃H or the group -CH₂CH(OH)SO₃Z';
Z' represents a cationic counterion derived from an alkali metal or alkaline-earth metal, such as sodium, an ammonium ion or an ion derived from an organic amine;
Ra' represents a C₁₀-C₃₀ alkyl or alkenyl group of an acid Ra'-COOH, which is preferably present in coconut oil or in hydrolysed linseed oil, or an alkyl group, especially a C₁₇ group and its iso form, or an unsaturated C₁₇ group.

The compounds of this type are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol® C2M Concentrate and the cocoamphodipropionate sold by the company Evonik Goldschmidt under the trade name Rewoteric AM KSF 40.

Ra"-NH-CH(Y")-(CH₂)ₙ-C(O)-NH-(CH₂)_{n'}-N(Rd)(Re) (IIa)

in which formula (IIa):
Y" represents the group -COOH, -COOZ", -CH₂CH(OH)SO₃H or the group -CH₂CH(OH)SO₃Z";
Rd and Re, independently of each other, represent a C₁-C₄ alkyl or hydroxyalkyl radical;
Z" represents a cationic counterion derived from an alkali metal or alkaline-earth metal, such as sodium, an ammonium ion or an ion derived from an organic amine;
Ra" represents a C₁₀-C₃₀ alkyl or alkenyl group of an acid Ra"-COOH which is preferably present in coconut oil or in hydrolysed linseed oil;
n and n' denote, independently of each other, an integer ranging from 1 to 3;
and mixtures of these compounds.

Among the compounds of formula (IIa), mention may be made of the compound classified in the CTFA dictionary under the name sodium diethylaminopropyl cocoaspartamide and sold by the company Chimex under the name Chimexane HB.

More preferentially, the amphoteric or zwitterionic surfactant(s) are chosen from cocoylbetaine, cocoylamidopropylbetaine and sodium cocoylamidoethyl-N-hydro xyethylaminopropionate.

The above amphoteric or zwitterionic surfactant(s) may be present in an amount ranging from 0.1% to 25% by weight, preferably from 0.5% to 15% by weight and better still from 1% to 10% by weight, relative to the total weight of the composition.

The term "cationic surfactant" means a surfactant that is positively charged when it is contained in the composition according to the invention. This surfactant may bear one or more positive permanent charges or may contain one or more functions that are cationizable in the composition according to the invention.

The cationic surfactant(s) are preferably chosen from optionally polyoxyalkylenated, primary, secondary or tertiary fatty amines, or salts thereof, and quaternary ammonium salts, and mixtures thereof.

The fatty amines generally comprise at least one C₈-C₃₀ hydrocarbon-based chain.

Examples of quaternary ammonium salts that may especially be mentioned include:
- those corresponding to the general formula (III) below: 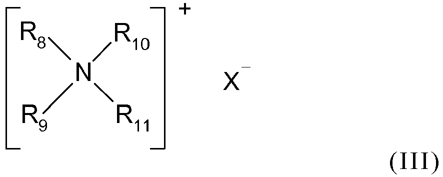 in which the groups R₈ to R₁₁, which may be identical or different, represent a linear or branched, saturated or unsaturated aliphatic group comprising from 1 to 30 carbon atoms, or an aromatic group such as aryl or alkylaryl, at least one of the groups R₈ to R₁₁ denoting a group comprising from 8 to 30 carbon atoms and preferably from 12 to 24 carbon atoms. The aliphatic groups may comprise heteroatoms especially such as oxygen, nitrogen, sulfur and halogens. The aliphatic groups are chosen, for example, from C₁-C₃₀ alkyl, C₂-C₃₀ alkenyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X⁻ is an anion chosen from the group of halides, phosphates, acetates, lactates, (C₁-C₄)alkyl sulfates, and (C₁-C₄)alkyl- or (C₁-C₄)alkylarylsulfonates.

Among the quaternary ammonium salts of formula (III), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, oleocetyldimethylhydroxyethylammonium salts, palmitylamidopropyltrimethylammonium salts, stearamidopropyltrimethylammonium salts and stearamidopropyldimethylcetearylammonium salts. It is particularly preferred to use the chloride salts of these compounds.
- quaternary ammonium salts of imidazoline, such as, for example, those of formula (IV) below: 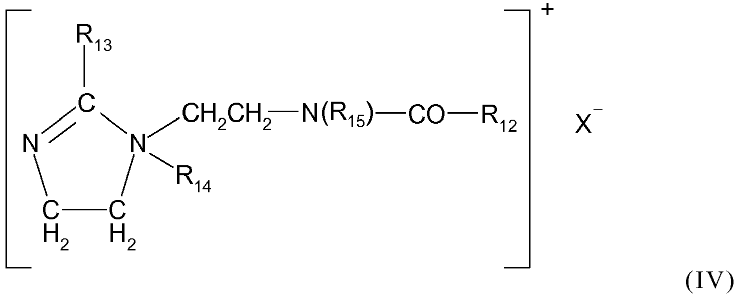 in which R₁₂ represents an alkenyl or alkyl group comprising from 8 to 30 carbon atoms, derived for example from tallow fatty acids, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X⁻ is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms. R₁₂ and R₁₃ preferably denote a mixture of alkenyl or alkyl groups containing from 12 to 21 carbon atoms, derived for example from tallow fatty acids, R₁₄ preferably denotes a methyl group, and R₁₅ preferably denotes a hydrogen atom. Such a product is sold, for example, under the name Rewoquat® W 75 by the company Rewo;
- quaternary diammonium or triammonium salts, in particular of formula (V): 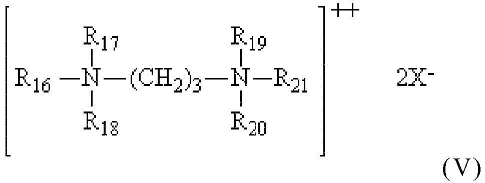
   in which R₁₆ denotes an alkyl radical comprising approximately from 16 to 30 carbon atoms, which is optionally hydroxylated and/or interrupted with one or more oxygen atoms, R₁₇ is chosen from hydrogen or an alkyl radical comprising from 1 to 4 carbon atoms or a group (R₁₆ₐ)(R₁₇ₐ)(R₁₈ₐ)N-(CH₂)₃,
   R₁₆ₐ, R₁₇ₐ, R₁₈ₐ, R₁₈, R₁₉, R₂₀ and R₂₁, which may be identical or different, being chosen from hydrogen and an alkyl radical comprising from 1 to 4 carbon atoms, and X⁻ is an anion chosen from the group of halides, acetates, phosphates, nitrates and methyl sulfates. Such compounds are, for example, Finquat CT-P, sold by the company Finetex (Quaternium 89), and Finquat CT, sold by the company Finetex (Quaternium 75),
- quaternary ammonium salts containing at least one ester function, such as those of formula (VI) below: 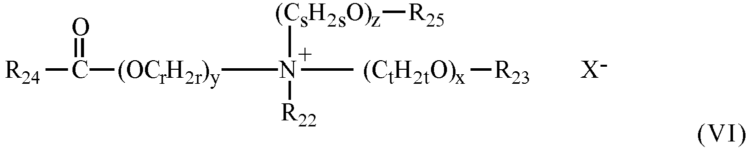 in which:
   R₂₂ is chosen from C₁-C₆ alkyl groups and C₁-C₆ hydroxyalkyl or dihydroxyalkyl groups;
   R₂₃ is chosen from:
- the group 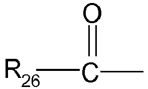
- groups R₂₇, which are linear or branched, saturated or unsaturated C₁-C₂₂ hydrocarbon-based groups,
- a hydrogen atom,
   R₂₅ is chosen from:
- the group 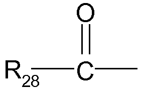
- groups R₂₉, which are linear or branched, saturated or unsaturated C₁-C₆ hydrocarbon-based groups,
- a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6;
   y is an integer ranging from 1 to 10;
   x and z, which may be identical or different, are integers ranging from 0 to 10;
   X⁻ is a simple or complex, organic or mineral anion;
   with the proviso that the sum x + y + z is from 1 to 15, that when x is 0 then R₂₃ denotes R₂₇, and that when z is 0 then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is a hydrocarbon-based group R₂₇, it may be long and contain from 12 to 22 carbon atoms, or may be short and contain from 1 to 3 carbon atoms.

When R₂₅ is an R₂₉ hydrocarbon-based group, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

Preferably, x and z, which may be identical or different, have values of 0 or 1.

Advantageously, y is equal to 1.

Preferably, r, s and t, which may be identical or different, are equal to 2 or 3, and even more particularly are equal to 2.

The anion X⁻ is preferably a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion compatible with the ammonium containing an ester function.

The anion X⁻ is even more particularly chloride or methyl sulfate.

Use is made more particularly, in the composition according to the invention, of the ammonium salts of formula (VI) in which:
R₂₂ denotes a methyl or ethyl group,
x and y are equal to 1;
z is equal to 0 or 1;
r, s and t are equal to 2;
R₂₃ is chosen from:
   - the group 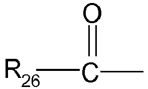
   - methyl, ethyl or C₁₄-C₂₂ hydrocarbon-based groups,
   - a hydrogen atom;
R₂₅ is chosen from:
   - the group 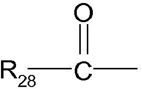
   - a hydrogen atom;
R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

The hydrocarbon-based groups are advantageously linear.

Mention may be made, for example, of the compounds of formula (VI) such as the diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by quaternization using an alkylating agent such as an alkyl (preferably methyl or ethyl) halide, a dialkyl (preferably methyl or ethyl) sulfate, methyl methanesulfonate, methyl para-toluenesulfonate, glycol chlorohydrin or glycerol chlorohydrin.

Such compounds are, for example, sold under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

It is also possible to use the ammonium salts containing at least one ester functional group that are described in Patents US-A-4 874 554 and US-A-4 137 180.

Use may be made of behenoylhydroxypropyltrimethylammonium chloride, provided by Kao under the name Quatarmin BTC 131.

Preferably, the ammonium salts containing at least one ester function contain two ester functions.

Among the quaternary ammonium salts containing at least one ester function, which can be used, it is preferred to use dipalmitoylethylhydroxyethylmethylammonium salts.

Among the above cationic surfactants, it is most particularly preferred to use those of formula (III) and those of formula (VI) and even more preferentially those of formula (III) and in particular cetyltrimethylammonium or behenyltrimethylammonium chlorides.

The above cationic surfactant(s) may be present in an amount ranging from 0.1% to 15% by weight, preferably from 0.5% to 10% by weight and better still from 1% to 5% by weight relative to the total weight of the composition.

The weight ratio according to the invention between the total amount of amphoteric or zwitterionic surfactant(s), on the one hand, and the total amount of cationic surfactant(s), on the other hand, is greater than or equal to 1.2.

Advantageously, the said ratio ranges from 1.2 to 10, preferably from 1.5 to 8 and better still from 2 to 4.

The sulfureous reducing agent(s) present in the composition according to the invention are preferably chosen from organic compounds comprising one or more mercapto groups (-SH), sulfites and sulfite derivatives.

The organic compounds comprising a mercapto group are preferably chosen from thioglycolic acid, thiolactic acid, cysteine, homocysteine, glutathione, thioglycerol, thiomalic acid, 2-mercaptopropionic acid, 3-mercaptopropionic acid, thiodiglycol, 2-mercaptoethanol, dithiothreitol, thioxanthine, thiosalicylic acid, thiodiglycolic acid, lipoic acid, N-acetylcysteine, and thioglycolic or thiolactic acid esters, and mixtures of these compounds.

The term "sulfite derivatives" essentially denotes bisulfites and sulfite diesters of formula R-O-SO₂-R', with R and R' denoting C₁-C₁₀ alkyl groups.

The sulfureous reducing agent(s) may be used especially in the form of salts, in particular alkali metal salts such as sodium and potassium salts, alkaline-earth metal salts, for example magnesium and calcium salts, ammonium salts, amine salts and amino alcohol salts.

In a particularly preferred manner, the sulfureous reducing agent(s) are chosen from thioglycolic acid and salts thereof, thiolactic acid and salts thereof, cysteine and salts thereof, alkali metal sulfites, alkali metal bisulfites, and precursors of these sulfites or bisulfites.

Even more preferentially, the sulfureous reducing agent(s) are chosen from thioglycolic acid, cysteine, sodium sulfite, sodium bisulfite and sodium metabisulfite.

The sulfureous reducing agent(s) are preferably present in an amount ranging from 0.1% to 10% by weight, preferably from 0.2% to 5% by weight and in particular from 1% to 5% by weight relative to the total weight of the composition.

The alkaline agent(s) used in the composition according to the invention may be any agent for increasing the pH of the composition in which it is present. The alkaline agent may be a Brönsted-Lowry or Lewis base. It may be mineral or organic.

In particular, the alkaline agent(s) may be chosen from:
a) aqueous ammonia,
b) alkanolamines such as mono-, di- and triethanolamine, isopropanolamine and 2-amino-2-methyl-1-propanol, and also derivatives thereof,
c) oxyethylenated and/or oxypropylenated ethylenediamines,
d) mineral or organic hydroxides,
e) alkali metal silicates such as sodium metasilicates,
f) amino acids, preferably basic amino acids, such as arginine, lysine, ornithine, citrulline and histidine,
g) carbonates and bicarbonates, particularly of a primary amine, secondary amine or tertiary amine, or of an alkali metal or alkaline-earth metal, or of ammonium, and
h) the compounds of formula (VII) below: 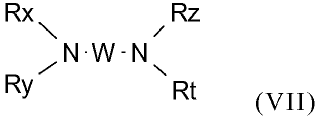
in which W is a C₁-C₆ alkylene residue optionally substituted with a hydroxyl group or a C₁-C₆ alkyl radical; Rx, Ry, Rz and Rt, which may be identical or different, represent a hydrogen atom or a C₁-C₆ alkyl, C₁-C₆ hydroxyalkyl or C₁-C₆ aminoalkyl radical.

Examples of such compounds of formula (VII) that may be mentioned include 1,3-diaminopropane, 1,3-diamino-2-propanol, spermine and spermidine.

The mineral or organic hydroxides are preferably chosen from hydroxides of an alkali metal, hydroxides of an alkaline-earth metal, for instance sodium hydroxide or potassium hydroxide, hydroxides of a transition metal, such as hydroxides of metals from groups III, IV, V and VI of the Periodic Table of the Elements, hydroxides of lanthanides or actinides, quaternary ammonium hydroxides and guanidinium hydroxide.

The hydroxide may be formed *in situ,* for instance guanidine hydroxide, by reacting calcium hydroxide and guanidine carbonate.

The preferred alkaline agents are in particular aqueous ammonia, ammonium carbonate, ammonium bicarbonate, arginine, monoethanolamine and 2-amino-2-methyl-1-propanol.

The alkaline agent(s) as defined previously may represent, for example, from 0.001% to 20% by weight, and preferably from 0.005% to 10% by weight, relative to the total weight of the composition according to the invention.

The concentration of alkaline agent(s) is especially adjusted as a function of the pH desired for the composition.

Preferably, the composition according to the invention has a pH ranging from 7 to 10 and more preferentially from 7 to 9.5.

The composition according to the invention may also comprise one or more nonionic surfactants and/or one or more anionic surfactants.

Examples of nonionic surfactants that may be used in the composition according to the invention are described, for example, in the Handbook of Surfactants by M.R. Porter, published by Blackie & Son (Glasgow and London), 1991, pp. 116-178. They are especially chosen from alcohols, α-diols and (C₁-C₂₄)alkylphenols, these compounds being polyethoxylated, polypropoxylated and/or polyglycerolated, and containing at least one fatty chain comprising, for example, from 8 to 18 carbon atoms, it being possible for the number of ethylene oxide and/or propylene oxide groups to especially range from 2 to 50, and for the number of glycerol groups to especially range from 2 to 30.

Mention may also be made of copolymers of ethylene oxide and propylene oxide, optionally oxyethylenated sorbitan fatty acid esters, sucrose fatty acid esters, polyoxyalkylenated fatty acid esters, polyoxyalkylenated fatty amides, optionally oxyalkylenated alkyl(poly)glucosides, alkylglucoside esters, derivatives of N-alkylglucamine and of N-acylmethylglucamine, aldobionamides, amine oxides and (poly)oxyalkylenated silicones.

The nonionic surfactants are more particularly chosen from monooxyalkylenated or polyoxyalkylenated and monoglycerolated or polyglycerolated nonionic surfactants, and alkyl(poly)glucosides. The oxyalkylene units are more particularly oxyethylene or oxypropylene units, or a combination thereof, preferably oxyethylene units.

Examples of preferred nonionic surfactants that may be mentioned include:
- oxyalkylenated (C₈-C₂₄)alkylphenols;
- saturated or unsaturated, linear or branched, oxyalkylenated C₈-C₄₀ alcohols;
- saturated or unsaturated, linear or branched, oxyalkylenated C₈-C₃₀ amides;
- esters of saturated or unsaturated, linear or branched, C₈-C₃₀ acids and of polyethylene glycols;
- saturated or unsaturated, oxyethylenated plant oils;
- condensates of ethylene oxide and/or of propylene oxide, alone or as mixtures;
- oxyethylenated and/or oxypropylenated silicones;
- alkyl(poly)glucosides.

As examples of monoglycerolated or polyglycerolated nonionic surfactants, monoglycerolated or polyglycerolated C₈-C₄₀ alcohols are preferably used.

In particular, the preferred monoglycerolated or polyglycerolated C₈-C₄₀ alcohols correspond to formula (VIII) below:

R₂₉O-[CH₂-CH(CH₂OH)-O]ₘ-H (VIII)

in which formula (VIII):
- R₂₉ represents a linear or branched C₈-C₄₀ and preferably C₈-C₃₀ alkyl or alkenyl radical; and
- m represents a number ranging from 1 to 30 and preferably from 1 to 10.

As examples of compounds of formula (VIII), mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol of formula (VIII) may represent a mixture of alcohols in the same way that the value of m represents a statistical value, which means that, in a commercial product, several species of polyglycerolated fatty alcohols may coexist in the form of a mixture.

The alkyl(poly)glycoside nonionic surfactant(s) may be represented by formula (IX) below:

R₃₀O-(R₃₁O)ₜ(G)ᵥ (IX)

in which:
R₃₀ represents a saturated or unsaturated, linear or branched alkyl group comprising from about 8 to 24 carbon atoms, or an alkylphenyl group in which the linear or branched alkyl group comprises from 8 to 24 carbon atoms;
R₃₁ represents an alkylene group containing from about 2 to 4 carbon atoms,
G represents a saccharide unit comprising from 5 to 6 carbon atoms,
t denotes a value ranging from 0 to 10 and preferably from 0 to 4, and
v denotes a value ranging from 1 to 15.

Preferably, the alkyl(poly)glycoside nonionic surfactant(s) correspond to formula (IX) in which:
R₃₀ denotes a linear or branched, saturated or unsaturated alkyl group containing from 8 to 18 carbon atoms,
G denotes glucose, fructose or galactose, preferably glucose,
t denotes a value ranging from 0 to 3, and is preferably equal to 0,
and R₃₁ and v are as defined previously.

The degree of polymerization of the alkyl(poly)glucoside nonionic surfactant(s), as represented, for example, by the index v in formula (IX), ranges on average from 1 to 15 and preferably from 1 to 4. This degree of polymerization more particularly ranges from 1 to 2 and better still from 1.1 to 1.5, on average.

The glycoside bonds between the saccharide units are of 1,6 or 1,4 type and preferably of 1,4 type.

Examples of compounds of formula (IX) that may especially be mentioned are the products sold by the company Cognis under the names Plantaren® (600 CS/U, 1200 and 2000) or Plantacare® (818, 1200 and 2000). Use may also be made of the products sold by the company SEPPIC under the names Triton CG 110 (or Oramix CG 110) and Triton CG 312 (or Oramix® NS 10), the products sold by the company BASF under the name Lutensol GD 70 or the products sold by the company Chem Y under the name AG10 LK.

Use may also be made, for example, of the 1,4- (C₈-C₁₆)alkylpolyglucoside as an aqueous solution at 53% by weight relative to the total weight of the solution, sold by Cognis under the reference Plantacare® 818 UP.

When they are present, the additional nonionic surfactant(s) may represent, for example, from 0.01% to 20% by weight and better still from 0.05% to 10% by weight relative to the total weight of the composition.

The term "anionic surfactant" means a surfactant comprising, as ionic or ionizable groups, only anionic groups. These anionic groups are chosen preferably from the groups CO₂H, CO₂⁻, SO₃H, SO3⁻, OSO₃H, OSO₃⁻, O₂PO₂H, O₂PO₂H⁻ and O₂PO₂²⁻.

The anionic surfactant(s) that may be used in the composition of the invention are chosen especially from alkyl sulfates, alkyl ether sulfates, alkylamido ether sulfates, alkylaryl polyether sulfates, monoglyceride sulfates, alkylsulfonates, alkylamide sulfonates, alkylarylsulfonates, α-olefin sulfonates, paraffin sulfonates, alkylsulfosuccinates, alkyl ether sulfosuccinates, alkylamide sulfosuccinates, alkyl sulfoacetates, acylsarcosinates, acylglutamates, alkylsulfosuccinamates, acylisethionates and N-acyltaurates, salts of alkyl monoesters and polyglycoside-polycarboxylic acids, acyllactylates, salts of D-galactoside uronic acids, salts of alkyl ether carboxylic acids, salts of alkyl aryl ether carboxylic acids, and salts of alkylamido ether carboxylic acids; or the non-salified forms of all of these compounds, the alkyl and acyl groups of all of these compounds containing from 6 to 24 carbon atoms and the aryl group denoting a phenyl group.

Some of these compounds may be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) are in salt form, they may be chosen especially from alkali metal salts such as the sodium or potassium salt and preferably the sodium salt, ammonium salts, amine salts and in particular amino alcohol salts, or alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Use is preferably made of (C₆-C₂₄)alkyl sulfates, (C₆-C₂₄)alkyl ether sulfates, which are optionally ethoxylated, comprising from 2 to 50 ethylene oxide units, and mixtures thereof, in particular in the form of alkali metal salts or alkaline-earth metal salts, ammonium salts or amino alcohol salts. More preferentially, the anionic surfactant(s) are chosen from (C₁₀-C₂₀)alkyl ether sulfates, and in particular sodium lauryl ether sulfate containing 2.2 mol of ethylene oxide.

When they are present, the additional anionic surfactant(s) may represent, for example, from 0.01% to 20% by weight and better still from 0.05% to 10% by weight relative to the total weight of the composition.

Advantageously, the compositions according to the invention may also contain one or more non-silicone fatty substances.

For the purposes of the present invention, the term "fatty substance" means an organic compound which, at room temperature (25°C) and at atmospheric pressure, is insoluble in water (i.e. it has a solubility in water of less than 1% by weight and preferably less than 0.5% by weight) and is soluble, under the same temperature and pressure conditions, in at least one organic solvent (for example ethanol, chloroform or benzene) to at least 1% by weight.

The term "non-silicone" refers to a fatty substance not comprising in its structure any Si-O-Si sequences.

The fatty substance(s) that may be used in the composition according to the present invention may be chosen especially from plant oils, animal oils, mineral oils, synthetic oils, fatty alcohols, and waxes other than fatty alcohols, and mixtures thereof.

Plant oils that may especially be mentioned include sweet almond oil, avocado oil, castor oil, olive oil, liquid jojoba wax, sunflower oil, wheatgerm oil, sesame oil, groundnut oil, grapeseed oil, soybean oil, rapeseed oil, safflower oil, coconut oil, corn oil, hazelnut oil, palm oil, apricot kernel oil, beauty-leaf oil, evening primrose oil, shea butter, rice bran oil, corn germ oil, passion flower oil and rye oil.

An animal oil that may especially be mentioned is perhydrosqualene.

Liquid paraffin or liquid petroleum jelly may especially be used as mineral oil.

Synthetic oils that may especially be mentioned include squalane, poly(α-olefins), for instance isododecane or isohexadecane, transesterified plant oils, fluoro oils and fatty esters.

The term "fatty esters" denotes compounds of formula RₐCOOR_{b} in which Rₐ represents a linear or branched, hydroxylated or non-hydroxylated, saturated or unsaturated higher acid residue, comprising from 4 to 29 carbon atoms, and R_{b} represents a linear or branched, saturated or unsaturated hydrocarbon-based chain containing from 3 to 30 carbon atoms, the total number of carbon atoms in the ester being greater than 10. Non-limiting examples that may especially be mentioned include Purcellin oil (stearyl octanoate), isopropyl myristate, isopropyl palmitate, butyl stearate, hexyl laurate, isononyl isononanoate, 2-ethylhexyl palmitate, 2-hexyldecyl laurate, 2-octadecyl palmitate, 2-octyldodecyl myristate, isostearyl neopentanoate and tridecyl neopentanoate.

The fluoro oils may be partially hydrocarbon-based and/or silicone-based fluoro oils, for instance those described in document JP-A-2-295 912.

The preferred fatty alcohols comprise, inter alia, myristyl alcohol, cetyl alcohol, stearyl alcohol, arachidyl alcohol, behenyl alcohol and erucyl alcohol.

For the purposes of the present invention, a wax is a lipophilic compound, which is solid at room temperature (about 25°C), with a reversible solid/liquid change of state, having a melting point greater than about 40°C, which may be up to 200°C, and having in the solid state anisotropic crystal organization. Animal and plant waxes comprise, as essential constituents, long-chain esters of carboxylic acids and of alcohols. In general, the size of the wax crystals is such that the crystals diffract and/or scatter light, giving the composition that comprises them a more or less opaque cloudy appearance. By bringing the wax to its melting point, it is possible to make it miscible with oils and to form a microscopically homogeneous mixture.

As waxes, other than fatty alcohols, that may be used in the composition according to the present invention, mention may be made of waxes of animal origin such as beeswax, spermaceti, lanolin wax and lanolin derivatives; plant waxes such as sunflower wax, rice wax, apple wax, carnauba wax, candelilla wax, ouricury wax, Japan wax, cocoa butter, cork fibre wax or sugar cane wax; mineral waxes, for example paraffin wax, petroleum jelly wax, lignite wax, microcrystalline waxes, ceresin or ozokerite, synthetic waxes such as polyethylene waxes and Fischer-Tropsch waxes, and mixtures thereof.

The non-silicone fatty substance(s) as described above, when they are present in the composition according to the invention, are preferably present in an amount ranging from 0.1% to 30% by weight, preferably from 1% to 20% by weight and better still from 5% to 15% by weight, relative to the total weight of the composition.

Advantageously also, the composition according to the invention may also comprise one or more swelling agents or emollients such as urea, glycols such as butylene glycol, hexylene glycol or propylene glycol, sorbitol, melamine, guanidine or glycerol.

Advantageously also, the composition according to the invention may comprise at least one surfactant, which is preferably nonionic, and/or at least one additional conditioning agent preferably chosen from silicones and/or cationic or amphoteric polymers.

As examples of silicones that may be used as conditioning agent(s) in the compositions of the invention, mention may be made of linear, cyclic, branched or unbranched, volatile or non-volatile silicones. These silicones may be in the form of oils, resins or gums, and may in particular be polyorganosiloxanes.

Organopolysiloxanes are defined in greater detail in Walter Noll's Chemistry and Technology of Silicones (1968), Academic Press. They may be volatile or non-volatile.

When they are volatile, the silicones are more particularly chosen from those with a boiling point of between 60°C and 260°C.

Conditioning agents that are most particularly preferred include cationic or amphoteric polymers especially such as Polyquaterniums 22, 6, 10, 11, 35 and 37, and hexadimethrine chloride.

The concentration of the additional conditioning agent(s) described above in the composition according to the invention may range from 0.01% to 10% by weight relative to the total weight of this composition, preferably from 0.05% to 5% by weight and even more preferentially from 0.1% to 3% by weight.

The composition in accordance with the invention may also comprise one or more cosmetic adjuvants other than the compounds described previously.

For example, it may comprise one or more standard additives that are well known in the art, such as agents for preventing hair loss, oxidizing agents, vitamins and provitamins including panthenol, sunscreens, mineral or organic pigments, sequestrants, plasticizers, solubilizers, acidifying agents, mineral or organic thickeners, antioxidants, hydroxy acids, nacreous agents, fragrances and preserving agents.

Needless to say, a person skilled in the art will take care to select this or these optional additional compound(s) such that the advantageous properties intrinsically associated with the composition according to the invention are not, or are not substantially, adversely affected by the envisaged addition(s).

The above additives may generally be present in an amount, for each of them, of between 0 and 20% by weight relative to the total weight of the composition.

According to a preferred embodiment, the composition according to the invention is non-colouring, i.e. it does not comprise any direct dyes or oxidation dyes.

A subject of the present invention is also the use of the composition according to the invention.

More particularly, a subject of the present invention is the use of a composition according to the invention for relaxing the curls of keratin fibres and/or for reducing the volume of keratin fibres, preferably of the hair.

In addition, this composition may advantageously be used for cleansing the hair, for straightening the hair or for a combined cleansing and straightening effect.

In particular, the composition according to the invention may be applied before or after a standard hair straightening treatment with or without fixing, so as to facilitate this treatment or reinforce its efficacy (straightening "booster" effect).

It may also be applied after a standard straightening treatment and especially alkaline straightening, so as to maintain the straightening of the hair, which makes it possible, for example, to space out the intervals of the straightening treatments generally performed with more aggressive compositions.

Another subject of the invention is a cosmetic process for treating keratin fibres, which comprises the application to the said wet or dry fibres, one or more times, of a composition as described above.

According to a preferred embodiment, such a process consists in applying to the keratin fibres the composition according to the present invention, and then in optionally rinsing the said fibres after an optional leave-on time.

According to a preferred embodiment, the process according to the invention comprises the following steps:
- applying the composition according to the invention to wet hair, and then
- leaving the composition to stand on the hair, adhering to a leave-on time ranging from 5 seconds to 60 minutes and preferably from 30 seconds to 15 minutes, at a temperature ranging from 20 to 230°C, preferably from 20 to 60°C and more preferably from 20 to 30°C, and then
- rinsing the hair with water.

The hair may then be dried, for example with a drying hood or a hairdryer, or may be left to dry in the open air.

It is also possible to perform a step of placing the hair under tension, for example by means of a brush, a comb or clips, at the moment when the hair is in contact with the composition according to the invention, or after rinsing the hair.

It is also possible to replace this step of placing under tension with a step of working the head of hair by hand or with a comb.

However, and this constitutes an advantage of the present invention, from the very first application of the composition, including an application without placing the hair under tension, a substantial reduction in the volume of the head of hair is observed. When the hair is curly, relaxation of the curls and/or better curl definition are also observed.

The process of the invention may be performed once.

According to one embodiment, the process according to the invention is repeated several times, until the desired level of straightening is obtained.

Thus, a subject of the present invention is also a process for relaxing hair curls and/or for reducing the volume of the hair, which consists in applying the composition according to the invention one or more times to the hair.

According to a preferred embodiment, the composition is applied several times, either consecutively or after a delay ranging from a few hours to a few days, each application being followed by rinsing, until the desired level of relaxation and/or of volume reduction is obtained.

The application of the composition according to the invention may be followed by an application of one or more rinse-out or leave-in hair compositions, optionally containing a reducing agent or an oxidizing agent.

According to a particular embodiment, the process according to the invention is a process for relaxing the curls of and/or for straightening keratin fibres, such as the hair, which comprises:
a) a step of applying to the keratin fibres the composition as described above; followed by
b) a step of applying to the keratin fibres a second composition (B) comprising one or more oxidizing agents and/or one or more reductones.

The second composition (B) used in this embodiment may comprise one or more oxidizing agents.

The said oxidizing agent(s) are preferably chosen from hydrogen peroxide, urea peroxide, alkali metal bromates or ferricyanides, peroxygenated salts, for instance persulfates, perborates, peracids and precursors thereof and alkali metal or alkaline-earth metal percarbonates, and most particularly hydrogen peroxide.

Preferably, the oxidizing agent(s) are not peroxygenated salts.

The oxidizing agent is preferably hydrogen peroxide.

The oxidizing agent(s) as defined previously may represent, when they are present, from 0.01% to 10% by weight and preferably from 0.1% to 5% by weight, relative to the total weight of composition (B).

The second composition (B) used in this embodiment may comprise one or more reductones.

In a manner known per se, the term "reductone" denotes a compound comprising an enediol structure -(HO)C=C(OH)- adjacent to a carbonyl group >C=O.

Thus, the reductone(s) that may be used in the present invention preferably have the general formula (X): 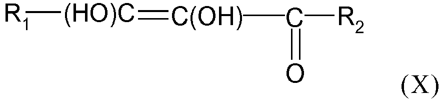 with R₁ and R₂, which may be identical or different, each denoting a group containing at least one carbon and/or oxygen atom, R₁ and R₂ possibly forming with the three carbon atoms of the compound of formula (X) a ring, which is preferably 5- or 6-membered, the additional constituent atoms of which consist of carbon and/or, oxygen atoms.

Preferably, R₁ and R₂ form with the three carbon atoms of the compound of formula (X) a ring containing 5 carbon and/or oxygen atoms.

The compound(s) of formula (X) may be in acid form, or in the form of salts, especially in the form of salts of alkali metals such as sodium and potassium, or salts of alkaline-earth metals such as calcium and magnesium, or in the form of esters, especially of C₈ to C₃₀ fatty acids.

In a particularly preferred manner, the compound of formula (X) is a lactone.

The reductone(s) may be chosen especially from reductic acid, ascorbic acid, erythorbic acid and isoascorbic acid, and the salts of these compounds, especially the sodium or potassium salts, ascorbyl palmitate, and mixtures of these compounds.

In a particularly preferred manner, the reductone(s) are chosen from ascorbic acid, erythorbic acid, and the salts of these compounds, especially the sodium or potassium salts.

The reductone(s) may represent, when they are present, from 0.1% to 10% by weight and preferably from 1% to 8% by weight relative to the total weight of composition (B).

When hydrogen peroxide is present in composition (B) used according to the invention, it may also comprise one or more hydrogen peroxide stabilizers.

The second composition (B) used according to the invention may comprise one or more pH regulators, which may be chosen from alkaline agents as described above and/or acidic agents.

The acidic agents that may be used according to the present invention may preferably be chosen from hydrochloric acid, (ortho)phosphoric acid, sulfuric acid, boric acid, and also carboxylic acids, for instance acetic acid, lactic acid or citric acid, or sulfonic acids.

Preferably, composition (B) used according to the invention has a pH ranging from 1 to 8.5, better still from 1 to 5 and more preferentially from 1.5 to 3.

Composition (B) may also contain one or more non-silicone fatty substances.

The non-silicone fatty substance(s) are as described previously for the composition according to the invention.

Composition (B) may also comprise one or more swelling agents or emollients as described previously for the composition according to the invention.

Composition (B) may also comprise at least one additional conditioning agent, preferably chosen from silicones and/or cationic or amphoteric polymers. These compounds are as described previously for the composition according to the invention.

Composition (B) may also comprise one or more cosmetic adjuvants as described previously for the composition according to the invention.

Composition (B) advantageously comprises water or a mixture of water and of one or more cosmetically acceptable solvents as described previously for the composition according to the invention.

According to a preferred embodiment, composition (B) is non-colouring, i.e. it does not comprise any direct dyes or oxidation dyes.

According to a preferred embodiment, the particular process according to the invention comprises the following steps:
- applying the composition according to the invention as described previously to wet or dry hair, preferably wet hair, and then
- leaving the composition to stand on the hair, adhering to a leave-on time ranging from 1 to 10 minutes and preferably from 2 to 5 minutes, at a temperature ranging from 20 to 230°C, preferably from 20 to 60°C and more preferably from 20 to 30°C, and then
- rinsing the hair with water,
- optionally rubbing dry and/or disentangling the hair, and then
- applying the second composition (B) as described previously, to the wet hair,
- leaving composition (B) to stand on the hair, adhering to a leave-on time ranging from 30 seconds to 10 minutes and preferably from 1 to 3 minutes, at a temperature ranging from 20 to 230°C, preferably from 20 to 60°C and more preferably from 20 to 30°C, and then
- optionally, rinsing the hair with water.

The hair may then be dried, for example by means of a drying hood or a hairdryer, or may be left in the open air.

It is also possible to perform a step of placing the hair under tension, for example by means of a brush, a comb or clips, at the moment when the hair is in contact with the composition according to the invention, or after rinsing the hair.

It is also possible to replace this step of placing under tension with a step of working the head of hair by hand or with a comb.

However, and this constitutes an advantage of the present invention, from the very first implementation of the process according to the invention, including an implementation without placing the hair under tension, a substantial reduction in the volume of the head of hair is observed. When the hair is curly, relaxation of the curls and/or better curl definition are also observed.

It is also found that the unpleasant odours that are given off during the straightening process, or that remain on the straightened hair, are reduced by means of performing the process according to the invention.

The process that is the subject of the present invention, may be performed once.

According to one embodiment, the process according to the invention is repeated several times, until the desired level of straightening is obtained.

According to a preferred embodiment, the process is repeated several times, either consecutively or after a delay ranging from a few hours to a few days.

In addition, this process may advantageously be performed for cleansing the hair, for straightening the hair or for a combined cleansing and straightening effect.

In particular, the process according to the invention may be performed before or after a standard hair straightening treatment with or without fixing, so as to facilitate this treatment or to reinforce its efficacy (straightening "booster" effect).

The process according to the invention may also be performed so as to maintain the straightening of the hair, which makes it possible, for example, to space out the intervals of the straightening treatments generally performed with more aggressive compositions.

A subject of the present invention is also a multi-compartment device or "kit" for relaxing the curls of and/or for straightening keratin fibres, which is suitable for performing the particular process as described above.

The kit according to the invention comprises:
- a first compartment containing a composition according to the invention as described above, and
- a second compartment containing a composition (B) as described above.

The compositions of this kit are packaged in separate compartments, which may be optionally accompanied by suitable identical or different application means, such as fine brushes, coarse brushes or sponges.

The abovementioned kit may also be equipped with means for dispensing the desired mixture on the hair, such as, for instance the device described in patent FR 2 586 913.

The examples that follow are given purely as illustrations of the present invention.

### EXAMPLES:

### Example 1: compositions according to the invention

Three foaming care compositions A, B and C in accordance with the invention were prepared from the ingredients indicated in the table below (in which the contents are indicated in grams of active material):

| Composition | A | B | C |
|---|---|---|---|
| Sodium N-cocoylamidoethyl-N-hydroxyethylaminopropionate⁽¹⁾ | 7.5 | 7.5 | 7.5 |
| Behenyltrimethylammonium chloride⁽²⁾ | 3.16 | 3.16 | 3.16 |
| Pregelatinized hydroxypropyl corn distarch phosphate | 3.5 | 3.5 | 3.5 |
| Cetylstearyl alcohol (50/50 C₁₆/C₁₈) | 7 | 7 | 7 |
| Polydimethylsiloxane containing aminoethyl aminopropyl groups, containing methoxy and/or hydroxyl and alpha-omega silanol functions⁽³⁾ | 1.1 | 1.1 | 1.1 |
| Thioglycolic acid | 2 | - | - |
| Sodium metabisulfite | - | 2 | - |
| Cysteine | - | - | 3 |
| Arginine | - | qs pH 7 | - |
| Monoethanolamine | - | - | qs pH 8 |
| Ammonium bicarbonate | qs pH 8 | - | - |
| Pentasodium pentetate | 0.16 | 0.16 | 0.16 |
| Water | qs 100 | qs 100 | qs 100 |

| | | | |
|---|---|---|---|
| (1) product Rewoteric AM KSF 40 sold by the company Evonik Goldschmidt (2) product Varisoft BT 85 sold by the company Evonik Goldschmidt (3) product Xiameter MEM-8299 Emulsion sold by the company Dow Corning | | | |

The above foaming care compositions were applied to heads of curly and/or voluminous hair, in the following manner: 20 to 30 g of care product were applied to the head of wet hair, i.e. hair that was moistened beforehand and rubbed dry using a terry towel.

The composition was then spread out carefully by massaging the head of hair, so as to work the composition into a lather.

After a leave-on time ranging from 2 to 5 minutes, the hair was rinsed thoroughly with water and then rubbed dry using a towel, disentangled with a comb and dried with a hairdryer.

The Applicant found that the hair was clean, and showed a marked reduction in the volume of the head of hair. As regards curly hair, the Applicant also found that the hair curls were more relaxed, with a better curl definition.

These effects may be obtained after only one application of the composition according to the invention, and without having to straighten the hair with a brush or a clip during drying.

The above treatment process may be repeated several times consecutively on the same head of hair. Enhanced effects are then observed.

### Example 2: use of compositions according to the invention in a particular two-step process

The invention was implemented using compositions A to C as described in Example 1 above and using the neutralizing compositions described below.

Two neutralizing compositions in accordance with the embodiment of the invention were prepared from the ingredients indicated in the table below (in which the contents are indicated in grams of active material):

| **Composition** | **D** | **E** |
|---|---|---|
| Diethylenetriaminepentaacetic acid | 0.06 | - |
| Tetrasodium phosphate | 0.02 | - |
| Disodium tin hexahydroxide | 0.04 | - |
| H₂O₂ | 0.6 | - |
| Ascorbic acid | - | 5 |
| pH regulator | qs pH 2.5 | - |
| Fragrance | 0.2 | - |
| Water | qs 100 g | qs 100 g |

Compositions A, B or C above were applied to heads of curly and/or voluminous hair, in the following manner: 30 to 40 g of composition were applied to the head of wet hair, i.e. hair that was moistened beforehand and rubbed dry using a terry towel.

The composition was then spread out carefully by massaging the head of hair, so as to work the composition into a lather.

After a leave-on time ranging from 2 to 5 minutes, the hair was rinsed thoroughly with water and then rubbed dry using a towel and disentangled with a comb.

Composition D or E was then applied to the head of hair, in the following manner: 30 to 40 g of composition were applied to the head of wet hair.

The composition was then spread out carefully by massaging the head of hair.

After a leave-on time of 2 minutes, the hair was rinsed thoroughly with water and then rubbed dry using a towel, disentangled with a comb and dried with a hairdryer.

The Applicant found that the hair showed a marked reduction in the volume of the head of hair. As regards curly hair, the Applicant also found that the hair curls were more relaxed, with a better curl definition.

These effects may be obtained after only one application of the composition according to the invention, and without having to straighten the hair with a brush or a clip during drying.

The Applicant furthermore found that the odour of the hair after treatment was greatly reduced when compared with the prior art treatments.

The above treatment process may be repeated several times consecutively on the same head of hair. Enhanced effects are then observed.

## Claims

1. Cosmetic composition comprising:
a) one or more amphoteric or zwitterionic surfactants chosen from the compounds having the structure (II) below:
Ra'-C(O)-NHCH₂CH₂-N(B)(B') (II)
in which formula (II):
B represents the group -CH₂-CH₂-O-X';
B' represents the group -(CH₂)_{z}Y', with z = 1 or 2;
X' represents the group -CH₂-COOH, -CH₂-COOZ', -CH₂CH₂-COOH or -CH₂CH₂-COOZ', or a hydrogen atom;
Y' represents the group -COOH, -COOZ', -CH₂CH(OH)SO₃H or the group -CH₂CH(OH)SO₃Z';
Z' represents a cationic counterion derived from an alkali metal or alkaline-earth metal, an ammonium ion or an ion derived from an organic amine;
Ra' represents a C₁₀-C₃₀ alkyl or alkenyl group of an acid Ra'-COOH,
b) one or more cationic surfactants,
c) one or more sulfureous reducing agents, and
d) one or more alkaline agents,
the weight ratio between the total amount of the amphoteric or zwitterionic surfactant(s), on the one hand, and the total amount of the cationic surfactant(s), on the other hand, being greater than or equal to 1.2.

2. Composition according to the preceding claim, **characterized in that** the said amphoteric or zwitterionic surfactant(s) is sodium cocoylamidoethyl-N-hydroxyethylaminopropionate.

3. Composition according to any one of the preceding claims, **characterized in that** the said amphoteric or zwitterionic surfactant(s) represent from 0.1% to 25% by weight, preferably from 0.5% to 15% by weight and better still from 1% to 10% by weight, relative to the total weight of the composition.

4. Composition according to any one of the preceding claims, **characterized in that** the said cationic surfactant(s) are chosen from:
- those corresponding to the general formula (III) below: 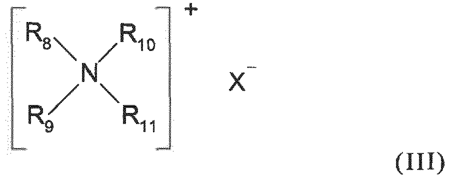
in which R₈ to R₁₁, which may be identical or different, represent a linear or branched, saturated or unsaturated aliphatic group comprising from 1 to 30 carbon atoms, or an aromatic group such as aryl or alkylaryl, at least one of the groups R₈ to R₁₁ denoting a group comprising from 8 to 30 carbon atoms and preferably from 12 to 24 carbon atoms;
X- is an anion chosen from the group of halides, phosphates, acetates, lactates, (C₁-C₄)alkyl sulfates, and (C₁-C₄)alkyl- or (C₁-C₄)alkylarylsulfonates;
- quaternary ammonium salts of imidazoline, and, more particularly, those of formula (IV) below: 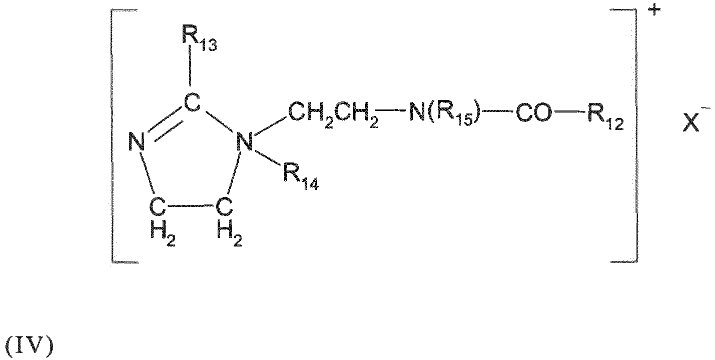
in which R₁₂ represents an alkenyl or alkyl group comprising from 8 to 30 carbon atoms, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group,
X- is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms;
- quaternary diammonium or triammonium salts, in particular of formula (V): 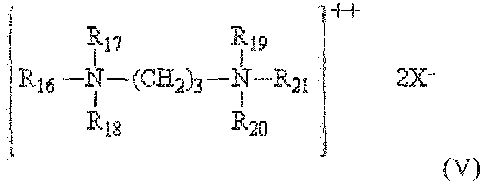
in which R₁₆ denotes an alkyl radical comprising approximately from 16 to 30 carbon atoms, which is optionally hydroxylated and/or interrupted with one or more oxygen atoms, R₁₇ is chosen from hydrogen or an alkyl radical comprising from 1 to 4 carbon atoms or a group (R₁₆ₐ)(R₁₇ₐ)(R₁₈ₐ)N-(CH₂)₃,
R₁₆ₐ, R₁₇ₐ, R₁₈ₐ, R₁₈, R₁₉, R₂₀ and R₂₁, which may be identical or different, being chosen from hydrogen and an alkyl radical comprising from 1 to 4 carbon atoms; and X- is an anion chosen from the group of halides, acetates, phosphates, nitrates and methyl sulfates;
- quaternary ammonium salts containing at least one ester function, such as those of formula (VI) below: 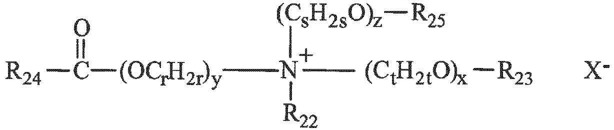 in which:
R₂₂ is chosen from C₁-C₆ alkyl groups and C₁-C₆ hydroxyalkyl or dihydroxyalkyl groups;
R₂₃ is chosen from:
- the group 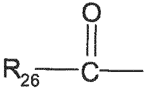
- groups R₂₇, which are linear or branched, saturated or unsaturated C₁-C₂₂ hydrocarbon-based groups,
- a hydrogen atom,
R₂₅ is chosen from:
- the group 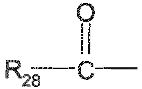
- groups R₂₉, which are linear or branched, saturated or unsaturated C₁-C₆ hydrocarbon-based groups,
- a hydrogen atom,
R₂₄, R₂₆ and R₂₈, which may be identical or different, are chosen from linear or branched, saturated or unsaturated C₇-C₂₁ hydrocarbon-based groups;
r, s and t, which may be identical or different, are integers ranging from 2 to 6;
y is an integer ranging from 1 to 10;
x and z, which may be identical or different, are integers ranging from 0 to 10;
X- is a simple or complex, organic or mineral anion;
with the proviso that the sum x + y + z is from 1 to 15, that when x is 0 then R₂₃ denotes R₂₇, and that when z is 0 then R₂₅ denotes R₂₉.

5. Composition according to any one of the preceding claims, **characterized in that** the said cationic surfactant(s) represent from 0.1% to 15% by weight, preferably from 0.5% to 10% by weight and better still from 1% to 5% by weight, relative to the total weight of the composition.

6. Composition according to any one of the preceding claims, **characterized in that** the weight ratio between the total amount of amphoteric or zwitterionic surfactant(s), on the one hand, and the total amount of cationic surfactant(s), on the other hand, ranges from 1.2 to 10, preferably from 1.5 to 8 and better still from 2 to 4.

7. Composition according to any one of the preceding claims, **characterized in that** the said sulfureous reducing agent(s) are chosen from organic compounds comprising one or more mercapto groups (-SH), sulfites and sulfite derivatives, preferably the said sulfureous reducing agent(s) are chosen from thioglycolic acid and salts thereof, thiolactic acid and salts thereof, cysteine and salts thereof, alkali metal sulfites, alkali metal bisulfites, and precursors of these sulfites or bisulfites.

8. Composition according to any one of the preceding claims, **characterized in that** the said sulfureous reducing agent(s) represent from 0.1% to 10% by weight and preferably from 0.2% to 5% by weight relative to the total weight of the composition.

9. Composition according to any one of the preceding claims, **characterized in that** the said alkaline agent(s) are chosen from:
a) aqueous ammonia,
b) alkanolamines and derivatives thereof,
c) oxyethylenated and/or oxypropylenated ethylenediamines,
d) mineral or organic hydroxides,
e) alkali metal silicates,
f) amino acids, which are preferably basic amino acids,
g) carbonates and bicarbonates, particularly of a primary amine, secondary amine or tertiary amine, or of an alkali metal or alkaline-earth metal, or of ammonium, and
h) the compounds of formula (VII) below: 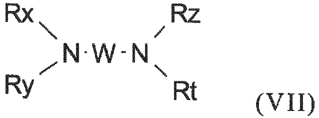
in which W is a C₁-C₆ alkylene residue optionally substituted with a hydroxyl group or a C₁-C₆ alkyl radical; Rx, Ry, Rz and Rt, which may be identical or different, represent a hydrogen atom or a C₁-C₆ alkyl, C₁-C₆ hydroxyalkyl or C₁-C₆ aminoalkyl radical.

10. Composition according to any one of the preceding claims, **characterized in that** it comprises at least one surfactant, which is preferably nonionic, and/or at least one additional conditioning agent preferably chosen from silicones and/or cationic or amphoteric polymers.

11. Process for relaxing the curls of and/or for straightening keratin fibres, such as the hair, **characterized in that** it comprises:
a) a step of applying to the keratin fibres the composition as defined in any one of Claims 1 to 10; followed by
b) a step of applying to the keratin fibres a second composition (B) comprising one or more oxidizing agents and/or one or more reductones.

12. Process according to Claim 11, **characterized in that** composition (B) comprises one or more oxidizing agents preferably chosen from hydrogen peroxide, urea peroxide, alkali metal bromates or ferricyanides and peroxygenated salts, and, more preferentially, the oxidizing agent is hydrogen peroxide.

13. Process according to Claim 12, **characterized in that** the said oxidizing agent(s) represent from 0.01% to 10% by weight and preferably from 0.1% to 5% by weight, relative to the total weight of composition (B).

14. Process according to any one of Claims 11 to 13, **characterized in that** composition (B) comprises one or more reductones, preferably chosen from the compounds of general formula (X): 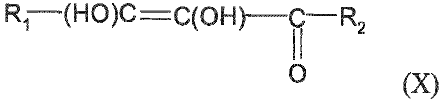
with R₁ and R₂, which may be identical or different, each denoting a group containing at least one carbon and/or oxygen atom, R₁ and R₂ possibly forming with the three carbon atoms of the compound of formula (X) a ring, which is preferably 5- or 6-membered, the additional constituent atoms of which consist of carbon and/or oxygen atoms,
the compound(s) of formula (X) being in acid form, or in the form of salts, especially in the form of salts of alkali metals or of alkaline-earth metals, or in the form of esters, especially of C₈ to C₃₀ fatty acids, and preferably the said reductone(s) are chosen from reductic acid, ascorbic acid, erythorbic acid and isoascorbic acid, and the salts of these compounds, especially the sodium or potassium salts, ascorbyl palmitate, and mixtures of these compounds.

15. Process according to Claim 14, **characterized in that** the said reductone(s) represent from 0.1% to 10% by weight and preferably from 1% to 8% by weight relative to the total weight of composition (B).

## Patentansprüche

1. Kosmetische Zusammensetzung, umfassend:
a) ein oder mehrere amphotere(s) oder zwitterionische(s) oberflächenaktive(s) Mittel, ausgewählt aus Verbindungen der nachstehenden Struktur (II):
Ra'-C(O)-NHCH₂CH₂-N(B)(B') (II)
worin in der Formel (II):
B die Gruppe -CH₂-CH₂-O-X' darstellt;
B' die Gruppe -(CH₂)_{z}Y' darstellt, wobei z = 1 oder 2 ist;
X' die Gruppe -CH₂-COOH, -CH₂-COOZ', -CH₂CH₂-COOH oder -CH₂CH₂-COOZ' oder ein Wasserstoffatom darstellt;
Y' die Gruppe -COOH, -COOZ', -CH₂CH(OH)SO₃H oder die Gruppe -CH₂CH(OH)SO₃Z' darstellt;
Z' ein kationisches Gegenion darstellt, das von einem Alkalimetall oder Erdalkalimetall, einem Ammoniumion oder einem aus einem organischen Amin stammenden Ion stammt;
Ra' eine C₁₀-C₃₀-Alkyl- oder -Alkenylgruppe einer Säure Ra'-COOH darstellt,
b) ein oder mehrere kationische(s) oberflächenaktive(s) Mittel,
c) ein oder mehrere schwefelhaltige(s) Reduktionsmittel und
d) ein oder mehrere alkalische(s) Mittel,
wobei das Gewichtsverhältnis zwischen der Gesamtmenge des bzw. der amphoteren oder zwitterionischen oberflächenaktiven Mittel(s) einerseits und der Gesamtmenge des bzw. der kationischen oberflächenaktiven Mittel(s) andererseits größer oder gleich 1,2 ist.

2. Zusammensetzung nach dem vorhergehenden Anspruch, **dadurch gekennzeichnet, dass** das bzw. die amphotere(n) oder zwitterionische(n) oberflächenaktive(n) Mittel Natriumcocoylamidoethyl-N-hydroxyethylaminopropionat ist bzw. sind.

3. Zusammensetzung nach einem der vorhergehenden Ansprüche, **dadurch gekennzeichnet, dass** das bzw. die amphotere(n) oder zwitterionische(n) oberflächenaktive(n) Mittel 0,1 bis 25 Gew.-%, vorzugsweise 0,5 bis 15 Gew.-%, besser noch 1 bis 10 Gew.-%, bezogen auf das Gesamtgewicht der Zusammensetzung, ausmacht bzw. ausmachen.

4. Zusammensetzung nach einem der vorhergehenden Ansprüche, **dadurch gekennzeichnet, dass** das bzw. die kationische(n) oberflächenaktive(n) Mittel ausgewählt ist bzw. sind aus:
- solchen, die der folgenden allgemeinen Formel (III) entsprechen: 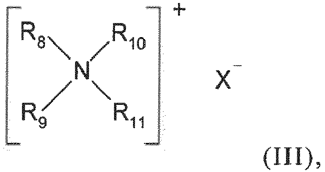
worin R₈ bis R₁₁, die gleich oder verschieden sein können, eine lineare oder verzweigte, gesättigte oder ungesättigte aliphatische Gruppe, die 1 bis 30 Kohlenstoffatome umfasst, oder eine aromatische Gruppe wie Aryl oder Alkylaryl darstellen, wobei mindestens eine der Gruppen R₈ bis R₁₁ eine Gruppe darstellt, die 8 bis 30 Kohlenstoffatome und vorzugsweise 12 bis 24 Kohlenstoffatome umfasst;
X⁻ ein Anion ist, ausgewählt aus der Gruppe der Halogenide, Phosphate, Acetate, Lactate, (C₁-C₄)-Alkylsulfate und (C₁-C₄)-Alkyl-oder (C₁-C₄)-Alkylarylsulfonate,
- quaternäre Ammoniumsalze von Imidazolin und insbesondere diejenigen der nachstehenden Formel (IV): 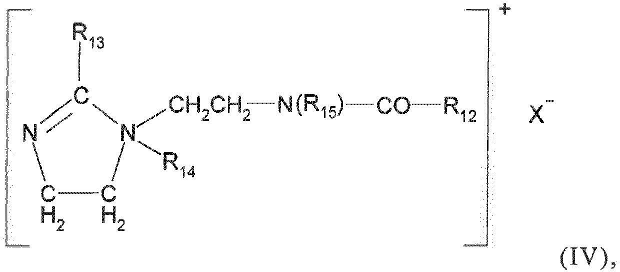
worin R₁₂ eine Alkenyl- oder Alkylgruppe darstellt, die 8 bis 30 Kohlenstoffatome umfasst, R₁₃ ein Wasserstoffatom, eine C₁-C₄-Alkylgruppe oder eine Alkyl- oder Alkenylgruppe darstellt, die 8 bis 30 Kohlenstoffatome umfasst, R₁₄ eine C₁-C₄-Alkylgruppe darstellt, R₁₅ ein Wasserstoffatom oder eine C₁-C₄-Alkylgruppe darstellt,
X⁻ ein Anion ist, ausgewählt aus der Gruppe der Halogenide, Phosphate, Acetate, Lactate, Alkylsulfate, Alkyl- oder Alkylarylsulfonate, worin die Alkyl- und Arylgruppen vorzugsweise 1 bis 20 Kohlenstoffatome bzw. 6 bis 30 Kohlenstoffatome umfassen;
- quaternäre Diammonium- oder Triammoniumsalze, insbesondere der Formel (V): 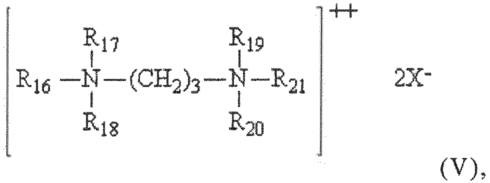
worin R₁₆ für einen Alkylrest steht, der etwa 16 bis 30 Kohlenstoffatome umfasst, der gegebenenfalls mit einem oder mehreren Sauerstoffatom(en) hydroxyliert und/oder unterbrochen ist, R₁₇ ausgewählt ist aus Wasserstoff oder einem Alkylrest, der 1 bis 4 Kohlenstoffatome oder eine Gruppe (R₁₆ₐ)(R₁₇ₐ)(R₁₈ₐ)N-(CH₂)₃ umfasst,
R₁₆ₐ, R₁₇ₐ, R₁₈ₐ, R₁₈, R₁₉, R₂₀ und R₂₁, die gleich oder verschieden sein können, aus Wasserstoff und einem Alkylrest, der 1 bis 4 Kohlenstoffatome umfasst, ausgewählt sind; und X- ein Anion ist, ausgewählt aus der Gruppe der Halogenide, Acetate, Phosphate, Nitrate und Methylsulfate;
- quaternäre Ammoniumsalze, die mindestens eine Esterfunktion enthalten, wie solche der nachstehenden Formel (VI): 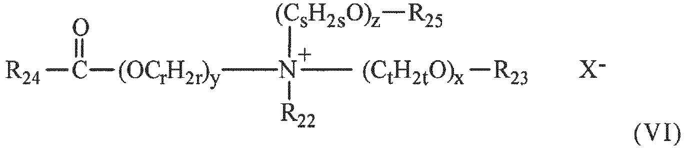 worin:
R₂₂ ausgewählt ist aus C₁-C₆-Alkylgruppen und C₁-C₆-Hydroxyalkyl- oder Dihydroxyalkylgruppen;
R₂₃ ausgewählt ist aus:
- der Gruppe 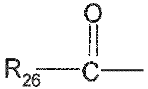
- den Gruppen R₂₇, die lineare oder verzweigte, gesättigte oder ungesättigte C₁-C₂₂-Kohlenwasserstoff-basierte Gruppen sind,
- einem Wasserstoffatom,
R₂₅ ausgewählt ist aus:
- der Gruppe 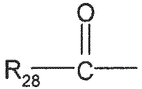
- den Gruppen R₂₉, die lineare oder verzweigte, gesättigte oder ungesättigte gesättigte C₁-C₆-Kohlenwasserstoff-basierte Gruppen sind,
- einem Wasserstoffatom,
R₂₄, R₂₆ und R₂₈, die gleich oder verschieden sein können, ausgewählt sind aus linearen oder verzweigten, gesättigten oder ungesättigten C₇-C₂₁-Kohlenwasserstoff-basierten Gruppen;
r, s und t, die gleich oder verschieden sein können, ganze Zahlen im Bereich von 2 bis 6 sind;
y eine ganze Zahl im Bereich von 1 bis 10 ist;
x und z, die gleich oder verschieden sein können, ganze Zahlen von 0 bis 10 sind;
X- ein einfaches oder komplexes organisches oder mineralisches Anion ist;
mit der Maßgabe, dass die Summe x + y + z von 1 bis 15 reicht, dass, wenn x gleich 0 ist, R₂₃ dann R₂₇ darstellt und wenn z gleich 0 ist, R₂₅ dann R₂₉ darstellt.

5. Zusammensetzung nach einem der vorhergehenden Ansprüche, **dadurch gekennzeichnet, dass** das bzw. die kationische(n) oberflächenaktive(n) Mittel 0,1 bis 15 Gew.-%, vorzugsweise 0,5 bis 10 Gew.-% und noch besser 1 bis 5 Gew.-%, bezogen auf das Gesamtgewicht der Zusammensetzung, ausmachen.

6. Zusammensetzung nach einem der vorhergehenden Ansprüche, **dadurch gekennzeichnet, dass** das Gewichtsverhältnis zwischen der Gesamtmenge an amphoterem/n oder zwitterionischem/n oberflächenaktivem/n Mittel(n) einerseits und der Gesamtmenge an kationischem/n oberflächenaktivem/n Mittel(n) andererseits von 1,2 bis 10, vorzugsweise von 1,5 bis 8 und noch besser von 2 bis 4 reicht.

7. Zusammensetzung nach einem der vorhergehenden Ansprüche, **dadurch gekennzeichnet, dass** das bzw. die schwefelhaltige(n) Reduktionsmittel aus organischen Verbindungen ausgewählt sind, die eine oder mehrere Mercaptogruppe(n) (-SH), Sulfite und Sulfitderivate umfassen, wobei vorzugsweise das bzw. die schwefelhaltige(n) Reduktionsmittel aus Thioglykolsäure und Salzen davon, Thiomilchsäure und Salzen davon, Cystein und Salzen davon, Alkalimetallsulfiten, Alkalimetallbisulfiten und Vorläufern dieser Sulfite oder Bisulfite ausgewählt ist bzw. sind.

8. Zusammensetzung nach einem der vorhergehenden Ansprüche, **dadurch gekennzeichnet, dass** das bzw. die schwefelhaltige(n) Reduktionsmittel 0,1 bis 10 Gew.-% und vorzugsweise 0,2 bis 5 Gew.-%, bezogen auf das Gesamtgewicht der Zusammensetzung, ausmachen.

9. Zusammensetzung nach einem der vorhergehenden Ansprüche, **dadurch gekennzeichnet, dass** das bzw. die alkalische(n) Mittel ausgewählt ist bzw. sind aus:
a) wässrigem Ammoniak,
b) Alkanolaminen und Derivaten davon,
c) oxyethylenierten und/oder oxypropylenierten Ethylendiaminen,
d) mineralischen oder organischen Hydroxiden,
e) Alkalimetallsilikaten,
f) Aminosäuren, bei denen es sich vorzugsweise um basische Aminosäuren handelt,
g) Carbonaten und Bicarbonaten, insbesondere eines primären Amins, sekundären Amins oder tertiären Amins, oder eines Alkali- oder Erdalkalimetalls, oder von Ammonium und
h) den Verbindungen der nachstehenden Formel (VII): 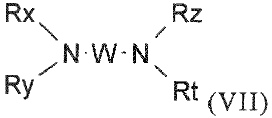
worin W ein C₁-C₆-Alkylenrest ist, der gegebenenfalls mit einer Hydroxylgruppe oder einem C₁-C₆-Alkylrest substituiert ist; Rx, Ry, Rz und Rt, die gleich oder verschieden sein können, ein Wasserstoffatom oder einen C₁-C₆-Alkyl-, C₁-C₆-Hydroxyalkyl- oder C₁-C₆-Aminoalkylrest darstellen.

10. Zusammensetzung nach einem der vorhergehenden Ansprüche, **dadurch gekennzeichnet, dass** sie mindestens ein oberflächenaktives Mittel, das vorzugsweise nicht-ionisch ist, und/oder mindestens ein zusätzliches Konditionierungsmittel, das vorzugsweise aus Silikonen und/oder kationischen oder amphoteren Polymeren ausgewählt ist, umfasst.

11. Verfahren zum Entspannen der Locken und/oder zum Glätten von Keratinfasern, wie Haar, **dadurch gekennzeichnet, dass** es umfasst:
a) einen Schritt, bei dem die Zusammensetzung nach einem der Ansprüche 1 bis 10 auf die Keratinfasern aufgebracht wird; gefolgt von
b) einen Schritt, bei dem eine zweite Zusammensetzung (B), die ein oder mehrere Oxidationsmittel und/oder ein oder mehrere Reduktone umfasst, auf die Keratinfasern aufgebracht wird.

12. Verfahren nach Anspruch 11, **dadurch gekennzeichnet, dass** die Zusammensetzung (B) ein oder mehrere Oxidationsmittel umfasst, die vorzugsweise aus Wasserstoffperoxid, Harnstoffperoxid, Alkalimetallbromaten oder -ferricyaniden und peroxygenierten Salzen ausgewählt sind, und stärker bevorzugt ist das Oxidationsmittel Wasserstoffperoxid.

13. Verfahren nach Anspruch 12, **dadurch gekennzeichnet, dass** das bzw. die Oxidationsmittel 0,01 bis 10 Gew.-% und vorzugsweise 0,1 bis 5 Gew.-%, bezogen auf das Gesamtgewicht der Zusammensetzung (B), ausmacht bzw. ausmachen.

14. Verfahren nach einem der Ansprüche 11 bis 13, **dadurch gekennzeichnet, dass** die Zusammensetzung (B) ein oder mehrere Reduktone umfasst, vorzugsweise ausgewählt aus den Verbindungen der allgemeinen Formel (X): 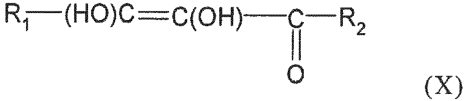
wobei R₁ und R₂, die gleich oder verschieden sein können, jeweils eine Gruppe bezeichnen, die mindestens ein Kohlenstoff- und/oder Sauerstoffatom enthält, wobei R₁ und R₂ womöglich mit den drei Kohlenstoffatomen der Verbindung der Formel (X) einen Ring bilden, der vorzugsweise 5- oder 6-gliedrig ist, dessen zusätzliche Bestandteilatome aus Kohlenstoff- und/oder Sauerstoffatomen bestehen,
wobei die Verbindung(en) der Formel (X) in Säureform oder in Form von Salzen, insbesondere in Form von Salzen von Alkalimetallen oder Erdalkalimetallen oder in Form von Estern, insbesondere von C₈- bis C₃₀-Fettsäuren, vorliegt bzw. vorliegen, und wobei das bzw. die Redukton(e) aus Reduktinsäure, Ascorbinsäure, Erythorbinsäure und Isoascorbinsäure und den Salzen dieser Verbindungen, insbesondere den Natrium- oder Kaliumsalzen, Ascorbylpalmitat und Gemischen dieser Verbindungen ausgewählt ist bzw. sind.

15. Verfahren nach Anspruch 14, **dadurch gekennzeichnet, dass** das bzw. die Redukton(e) 0,1 bis 10 Gew.-% und vorzugsweise 1 bis 8 Gew.-%, bezogen auf das Gesamtgewicht der Zusammensetzung (B), ausmacht bzw. ausmachen.

## Revendications

1. Composition cosmétique, comprenant:
a) un ou plusieurs tensioactif(s) amphotère(s) ou zwittérionique(s) choisis parmi les composés de structure (II) suivante :
Ra'-C(O)-NHCH₂CH₂-N(B)(B') (II)
Formule (II) dans laquelle :
B représente le groupe -CH₂-CH₂-O-X' ;
B' représente le groupe -(CH₂)_{z}Y', avec z = 1 ou 2 ;
X' représente le groupe -CH₂-COOH, -CH₂-COOZ', -CH₂-CH₂-COOH, -CH₂CH₂-COOZ', ou un atome d'hydrogène ;
Y' représente le groupe -COOH, -COOZ', -CH₂-CH(OH)-SO₃H ou le groupe -CH₂-CH(OH)-SO₃-Z' ;
Z' représente un contre ion cationique issu d'un métal alcalin ou alcalinoterreux, un ion ammonium ou un ion issu d'une amine organique ;
Ra' représente un groupe alkyle ou alcényle en C₁₀-C₃₀ d'un acide Ra'-COOH,
b) un ou plusieurs tensioactif(s) cationique(s),
c) un ou plusieurs agent(s) réducteur(s) soufré(s), et
d) un ou plusieurs agent(s) alcalin(s),
le rapport pondéral entre la quantité totale du ou des tensioactif(s) amphotère(s) ou zwittérionique(s) d'une part et la quantité totale du ou des tensioactif(s) cationique(s) d'autre part étant supérieur ou égal à 1,2.

2. Composition selon la revendication précédente, **caractérisée en ce que** le ou lesdits tensioactif(s) amphotère(s) ou zwittérionique(s) est le cocoyl amidoéthyl-N-hydroxyéthyl aminoproprionate de sodium.

3. Composition selon l'une quelconque des revendications précédentes, **caractérisée en ce que** le ou lesdits tensioactif(s) amphotère(s) ou zwittérionique(s) représentent de 0,1 à 25 % en poids, de préférence de 0,5 à 15 % en poids, et mieux encore de 1 à 10 % en poids, par rapport au poids total de la composition.

4. Composition selon l'une quelconque des revendications précédentes, **caractérisée en ce que** le ou lesdits tensioactif(s) cationique(s) sont choisis parmi :
- ceux répondant à la formule générale (III) suivante : 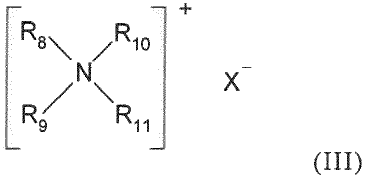
avec R₈ à R₁₁, identiques ou différents, représentent un groupe aliphatique, linéaire ou ramifié, saturé ou insaturé, comportant de 1 à 30 atomes de carbone, ou un groupe aromatique tel que aryle ou alkylaryle, au moins un des groupes R₈ à R₁₁ désignant un groupe comportant de 8 à 30 atomes de carbones, de préférence de 12 à 24 atomes de carbone ;
X⁻ est un anion choisi dans le groupe des halogénures, phosphates, acétates, lactates, alkyl(C₁-C₄)sulfates, alkyl(C₁-C₄)- ou alkyl(C₁-C₄)aryl-sulfonates ;
- les sels d'ammonium quaternaire de l'imidazoline, et plus particulièrement ceux de formule (IV) suivante : 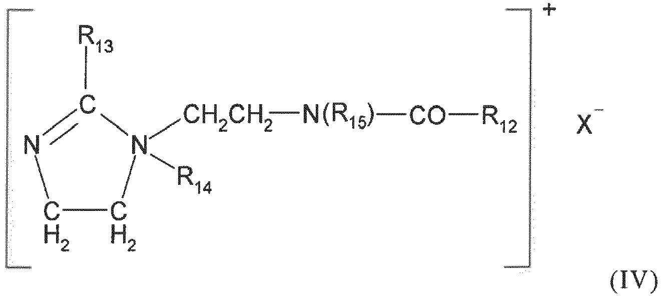
dans laquelle R₁₂ représente un groupe alcényle ou alkyle comportant de 8 à 30 atomes de carbone, R₁₃ représente un atome d'hydrogène, un groupe alkyle en C₁-C₄ ou un groupe alcényle ou alkyle comportant de 8 à 30 atomes de carbone, R₁₄ représente un groupe alkyle en C₁-C₄, R₁₅ représente un atome d'hydrogène, un groupe alkyle en C₁-C₄,
X⁻ est un anion choisi dans le groupe des halogénures, phosphates, acétates, lactates, alkylsulfates, alkyl- ou alkylaryl-sulfonates dont les groupements alkyl et aryl comprennent de préférence respectivement de 1 à 20 atomes de carbone et de 6 à 30 atomes de carbone ;
- les sels de di ou de triammonium quaternaire, en particulier de formule (V) : 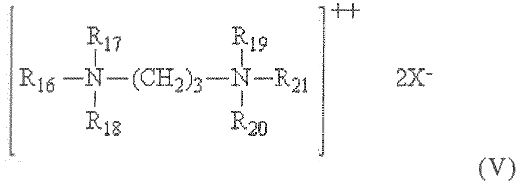
dans laquelle R₁₆ désigne un radical alkyle comportant environ de 16 à 30 atomes de carbone éventuellement hydroxylé et/ou interrompu par un ou plusieurs atomes d'oxygène, R₁₇ est choisi parmi l'hydrogène ou un radical alkyle comportant de 1 à 4 atomes de carbone ou un groupe (R₁₆ₐ)(R₁₇ₐ)(R₁₈ₐ)N-(CH₂)₃,
R₁₆ₐ, R₁₇ₐ, R₁₈ₐ, R₁₈, R₁₉, R₂₀ et R₂₁, identiques ou différents, étant choisis parmi l'hydrogène ou un radical alkyle comportant de 1 à 4 atomes de carbone ; et X- est un anion choisi dans le groupe des halogénures, acétates, phosphates, nitrates et méthylsulfates ;
- les sels d'ammonium quaternaire contenant au moins une fonction ester, tels que ceux de formule (VI) suivante : 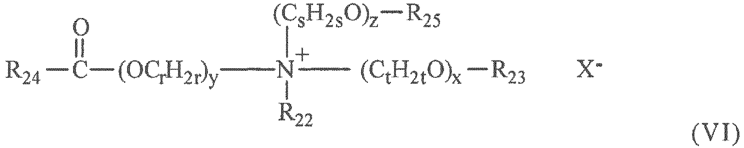 dans laquelle :
R₂₂ est choisi parmi les groupes alkyles en C₁-C₆ et les groupes hydroxyalkyles ou dihydroxyalkyles en C₁-C₆ ;
R₂₃ est choisi parmi :
- le groupe 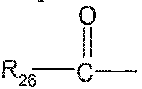
- les groupes R₂₇ qui sont des groupes hydrocarbonés en C₁-C₂₂, linéaires ou ramifiés, saturés ou insaturés,
- l'atome d'hydrogène,
R₂₅ est choisi parmi :
- le groupe 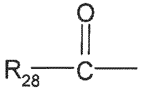
- les groupes R₂₉ qui sont des groupes hydrocarbonés en C₁-C₆, linéaires ou ramifiés, saturés ou insaturés,
- l'atome d'hydrogène,
R₂₄, R₂₆ et R₂₈, identiques ou différents, sont choisis parmi les groupes hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ; sous réserve que la somme x + y + z vaut de 1 à 15, que lorsque x vaut 0 alors R₂₃ désigne R₂₇ et que lorsque z vaut 0 alors R₂₅ désigne R29.

5. Composition selon l'une quelconque des revendications précédentes, **caractérisée en ce que** le ou lesdits tensioactif(s) cationique(s) représentent de 0,1 à 15 % en poids, de préférence de 0,5 à 10 % en poids, et mieux encore de 1 à 5 % en poids, par rapport au poids total de la composition.

6. Composition selon l'une quelconque des revendications précédentes, **caractérisée en ce que** le rapport pondéral entre la quantité totale du ou des tensioactif(s) amphotère(s) ou zwittérionique(s) d'une part et la quantité totale du ou des tensioactif(s) cationique(s) d'autre part varie de 1,2 à 10, de préférence de 1,5 à 8, et mieux encore de 2 à 4.

7. Composition selon l'une quelconque des revendications précédentes, **caractérisée en ce que** le ou lesdits agent(s) réducteur(s) soufré(s) sont choisis parmi les composés organiques comportant un ou plusieurs groupements mercapto (-SH), les sulfites, et les dérivés de sulfites, de préférence le ou les dits agent(s) réducteur(s) soufré(s) sont choisis parmi l'acide thioglycolique et ses sels, l'acide thiolactique et ses sels, la cystéine et ses sels, les sulfites de métaux alcalins, les bisulfites de métaux alcalins, et les précurseurs de ces sulfites ou bisulfites.

8. Composition selon l'une quelconque des revendications précédentes, **caractérisée en ce que** le ou les dits agent(s) réducteur(s) soufré(s) représentent de 0,1 à 10 % en poids, de préférence de 0,2 à 5 % en poids, par rapport au poids total de la composition.

9. Composition selon l'une quelconque des revendications précédentes, **caractérisée en ce que** le ou lesdits agent(s) alcalin(s) sont choisis parmi:
a) l'ammoniaque,
b) les alcanolamines ainsi que leurs dérivés,
c) les éthylènediamines oxyéthylénées et/ou oxypropylénées,
d) les hydroxydes minéraux ou organiques,
e) les silicates de métaux alcalins,
f) les acides aminés de préférence basiques,
g) les carbonates et bicarbonates particulièrement d'amine primaire, secondaire ou tertiaire, de métal alcalin ou alcalino-terreux, ou d'ammonium, et
h) les composés de formule (VII) suivante : 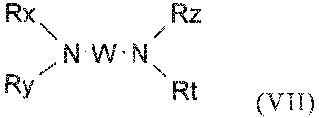 dans laquelle W est un reste alkylène en C₁-C₆ éventuellement substitué par un groupement hydroxyle ou un radical alkyle en C₁-C₆; Rx, Ry, Rz et Rt, identiques ou différents, représentent un atome d'hydrogène, un radical alkyle en C₁-C₆, hydroxyalkyle en C₁-C₆, ou aminoalkyle en C₁-C₆.

10. Composition selon l'une quelconque des revendications précédentes, **caractérisée en ce qu'**elle comprend au moins un tensioactif de préférence non-ionique et/ou au moins un agent de conditionnement additionnel choisi de préférence parmi les silicones et/ou les polymères cationiques ou amphotères.

11. Procédé de détente de boucles et/ou de lissage des fibres kératiniques, telles que les cheveux, **caractérisé en ce qu'**il comprend :
a) une étape d'application sur les fibres kératiniques de la composition telle que définie dans l'une quelconque des revendications 1 à 10 ; suivie de
b) une étape d'application sur les fibres kératiniques d'une seconde composition (B) comprenant un ou plusieurs agent(s) oxydant(s) et/ou une ou plusieurs réductone(s).

12. Procédé selon la revendication 11, **caractérisé en ce que** la composition (B) comprend un ou plusieurs agent(s) oxydant(s), de préférence choisis parmi le peroxyde d'hydrogène, le peroxyde d'urée, les bromates ou ferricyanures de métaux alcalins, les sels peroxygénés, et plus préférentiellement l'agent oxydant est le peroxyde d'hydrogène.

13. Procédé selon la revendication 12, **caractérisé en ce que** le ou lesdits agent(s) oxydant(s) représentent de 0,01 à 10 % en poids, de préférence de 0,1 à 5 % en poids, par rapport au poids total de la composition (B).

14. Procédé selon l'une quelconque des revendications 11 à 13, **caractérisé en ce que** la composition (B) comprend une ou plusieurs réductone(s), de préférence choisies parmi les composés de formule générale (X) : 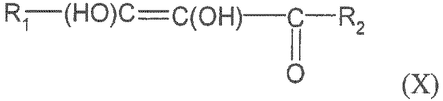
avec R₁ et R₂, identiques ou différents, désignant chacun un groupement contenant au moins un atome de carbone et/ou d'oxygène, R₁ et R₂ pouvant former avec les trois atomes de carbone du composé de formule (X) un cycle de préférence à 5 ou 6 chaînons, dont les atomes constitutifs supplémentaires sont constitués par des atomes de carbone et/ou d'oxygène,
le ou les composés de formule (X) se présentant sous forme acide, ou sous forme de sels, notamment sous forme de sels de métaux alcalins ou de métaux alcalino-terreux, ou sous forme d'esters notamment d'acides gras en C₈ à C₃₀, et de préférence la ou lesdites réductone(s) sont choisies parmi l'acide réductique, l'acide ascorbique, l'acide érythorbique ou isoascorbique, et les sels de ces composés, notamment les sels de sodium ou de potassium, le palmitate d'ascorbyle, et les mélanges de ces composés.

15. Procédé selon la revendication 14, **caractérisé en ce que** la ou lesdites réductone(s) représentent de 0,1 à 10 % en poids, de préférence de 1 à 8 % en poids, par rapport au poids total de la composition (B).
